# EUROPÄISCHE PATENTSCHRIFT

(11) **EP 2 015 049 B1**
(45) Veröffentlichungstag und Bekanntmachung des Hinweises auf die Patenterteilung: **21.01.2015**
(21) Anmeldenummer: 08010689.1
(22) Anmeldetag: 12.06.2008
(51) Int. Cl.: G01N 1/22, G01N 33/20

(54) **Vorrichtung zum Sammeln von Gasen in Metallschmelzen und entsprechendes Messverfahren**
Device for gathering gases in metal melts and corresponding measuring method
Dispositif de collecte de gaz dans des métaux en fusion et procédé de mesure

(30) Priorität: 10.07.2007 DE 102007032436
(43) Veröffentlichungstag der Anmeldung: 14.01.2009
(60) Teilanmeldung: 14171474.1
(73) Patentinhaber: Heraeus Electro-Nite International N.V., 3530 Houthalen (BE)
(72) Erfinder: Gerits, Erik, 3600 Genk (BE); Verstreken, Paul Clement, 3200 Aarschot (BE); Swennen, Jos, 3670 Meeuwen-Gruitrode (BE); Aegten, Jozef Theodor, 3950 Bocholt (BE)
(74) Vertreter: Kühn, Hans-Christian

(56) Entgegenhaltungen:
- EP-A2- 0 238 054
- EP-A2- 0 642 019
- DE-A1- 4 135 510
- DE-A1-102005 011 181
- US-A- 2 861 450
- US-A- 3 529 459
- US-A- 3 886 444
- US-A- 3 950 992
- US-A- 5 850 034
- US-B1- 6 216 526

## Beschreibung

Die Erfindung betrifft eine Vorrichtung zum Sammeln von Gasen in Metallschmelzen mit einem einen Sammelkörper aufweisenden Eintauchende, einer an dem Eintauchende mündenden Gaszuleitung und einer Gasableitung für die den Sammelkörper durchdringenden Gase, wobei der Gassammelkörper eine am Eintauchende angeordnete Stirnseite und Seitenwände aufweist.

Derartige Vorrichtungen sind beispielsweise aus DE 10 2005 011 181 A1 oder aus EP 307 430 B1 bekannt. In derartigen Vorrichtungen werden Gase aus einer Metallschmelze aufgesammelt und einer Messeinrichtung zugeführt, so dass der in der Metallschmelze enthaltene Gehalt an bestimmten Gasen gemessen werden kann. Dazu wird eine Gaszuleitung für die Zuleitung von Referenzgas bzw. Trägergas in die Metallschmelze durch den Gassammelkörper hindurch und an seiner Stirnseite aus ihm herausgeführt. Mit Hilfe der Gaszuleitung wird Referenzgas in die Metallschmelze eingeblasen. Das Referenzgas reichert sich mit in der Metallschmelze vorhandenen Gasen an oder, nach einer anderen Verfahrensweise, weist das Referenzgas eine höhere Konzentration des zu messenden Gases auf als die Metallschmelze, so dass das entstehende Gasgemisch eine geringere Konzentration des zu messenden Gasbestandteils aufweist als das Referenzgas. Das entstehende Gasgemisch wird von dem Gassammelkörper aufgenommen, durch die Gasableitung der Messeinrichtung zugeführt und ausgewertet. Im Detail ist das Messverfahren beispielsweise in EP 307 430 B1 beschrieben. Auch in EP 563 447 A1 sind derartige Messverfahren beschrieben.
Ähnliche Vorrichtungen sind aus US 6,216,526 B1 und aus EP 295 798 A1 bekannt.

Aufgabe der vorliegenden Erfindung ist es, die bekannten Gassammelvorrichtungen zu verbessern und die Effizienz des Sammelvorganges sowie des Messverfahrens zu erhöhen.

Die Aufgabe wird durch die Merkmale des unabhängigen Anspruchs gelöst. Vorzugsweise Ausgestaltungen ergeben sich aus den Unteransprüchen. Dadurch, dass zumindest ein Teil des Gassammelkörpers eine gasundurchlässige Schicht aufweist, ist es möglich, einen größeren Teil der Gase durch den Gassammelkörper aufzunehmen und der Gasableitung und damit der Messeinrichtung zuzuführen, da die in den Gassammelkörper eindringenden Gase den Gassammelkörper zumindest im Wesentlichen nicht mehr außerhalb der Gasableitung verlassen können, so dass ein deutlich größerer Anteil der in dem Gassammelkörper aufgenommenen Gase der Messeinrichtung zugeführt werden kann. Dadurch wird die Messung einfacher, schneller und letztendlich auch genauer.

Zweckmäßig ist es, dass mindestens ein Teil der äußeren Seitenwände eine gasundurchlässige Schicht aufweist. Der Gassammelkörper selbst kann an seiner Stirnseite einen bereits aus dem Stand der Technik (s. oben) bekannten Hohlraum aufweisen. In diesem Hohlraum sammeln sich zunächst die aus der Schmelze kommenden Gase. Sie dringen dann in den Gassammelkörper ein, da sie aus dem Hohlraum nicht anders entweichen können. Durch die seitliche Abschirmung durch die gasundurchlässige Schicht können die Gase nur in die Gasableitung entweichen. Dazu kann die gasundurchlässige Schicht an der Oberfläche die Seitenwände des Gassammelkörpers angeordnet sein. Vorteilhaft ist es, dass die Schicht aus mindesten zwei aufeinander angeordneten Teilschichten gebildet ist. Die untere, dem Inneren des Gassammelkörpers zugewandte Teilschicht kann aus Metall, insbesondere aus einem Metall mit höherem Schmelzpunkt als Eisen gebildet sein. Als Metalle kommen insbesondere in Frage Molybdän, Titan, Vanadium, Chrom, Niob oder eine Legierung mit mindestens einem dieser Metalle. Die untere, innere Teilschicht ist gasdicht. Auf ihr kann eine äußere, dem Inneren des Gassammelkörpers abgewandte Teilschicht aus Keramik aufgebracht sein. Diese kann als Schutzschicht für die zwischen ihr und dem Gassammelkörper angeordnete untere Teilschicht aus Metall wirken. Die äußere Teilschicht kann vorzugsweise aus Oxidkeramik oder einem Silikat, insbesondere aus Zirkoniumdioxid, Aluminiumoxid, Chromdioxid, Zirkoniumsilikat, Aluminiumsilikat oder Spinell gebildet sein.

Der Gassammelkörper kann mit der Schicht nahezu vollständig umgeben sein, wobei lediglich der stirnseitige Gaseintritt in den Gassammelkörper und der Zugang zur Gasableitung aus dem Gassammelkörper unbeschichtet sind. Sinnvoll ist es, die gesamte Stirnseite des Gassammelkörpers beschichtungsfrei zu lassen, oder auch nur die Oberfläche des stirnseitigen Hohlraumes des Gassammelkörpers. Vorzugsweise ist mindestens eine der Teilschichten plasmagespritzt.

Zweckmäßigerweise kann der Gassammelkörper eine zylindrische oder konische Seitenwand aufweisen. Die Gasableitung ist vorzugsweise an der der Stirnseite gegenüberliegenden Rückwand des Gassammelkörpers angeordnet. Die Gasableitung kann beispielsweise an einem Gasanschlussstutzen oder in einer Öffnung des Gassammelkörpers angeordnet sein.

Die Vorrichtung wird erfindungsgemäß zum Messen des Gasgehaltes in einer Metallschmelze verwendet. Es sind Messungen zum Beispiel in den unterschiedlichsten Stahlschmelzen möglich. Der Gassammelkörper selbst ist undurchlässig für die Metallschmelze, aber sehr gut gasdurchlässig und aufnahmefähig für die zu messenden Gase.

Ein entsprechendes Messverfahren zum Messen eines Gasgehaltes in einer Metallschmelze, wobei Gas in die Metallschmelze eingeleitet wird, dort in einen Gasaustausch mit in der Metallschmelze enthaltenem Gas tritt und anschließend aufgenommen und einer Messeinrichtung zur Auswertung zugeführt wird, wobei mindestens zwei unterschiedliche Gase in die Metalischmelze eingeleitet und ausgewertet werden, wobei beide Gase jeweils ein Trägergas aufweisen und gegebenenfalls eine Beimischung aus einem Gas, dessen Anteil in der Metallschmelze bestimmt werden soll erfolgt dadurch, dass die Konzentration des beigemischten Gases entweder in jedem Fall der Gaseinleitung unterhalb oder in jedem Fall der Gaseinleitung oberhalb der Konzentration des zu messenden Gases in der Metallschmelze liegt. Dabei wird von der vermutlichen Gaskonzentration in der Metallschmelze ausgegangen und für das einzuleitende Gas eine Konzentration entweder deutlich unter oder deutlich über der erwarteten Konzentration in der Metallschmelze gewählt. Es erfolgt dann in der Metallschmelze bei beiden Gasen entweder eine Absorption oder eine Desorption des zu messenden Gases. Es wird also mit zwei (oder mehr) Gasen gemessen, die unabhängig voneinander sind. Dabei können die gleichen oder unterschiedliche Trägergase verwendet werden. Die in die Schmelze eingeleiteten Gase nehmen Gas aus der Schmelze auf, wenn die Konzentration des zu bestimmenden Gases in der Metallschmelze höher ist als die Konzentration dieses Gases in dem eingeleiteten Gas, so dass als eingeleitetes Gas auch reines Trägergas verwendet werden und die Konzentration des zu messenden Gases in dem eingeleiteten Gas Null sein kann. Im umgekehrten Fall nimmt die Metallschmelze Gas aus dem eingeleiteten Gas auf, da in jedem Fall naturgemäß ein Gleichgewicht angestrebt wird. Zur Messung kann der Umstand genutzt werden, dass die Absorptions- und die Desorptionscharakteristiken von unterschiedlichen Gasen in Metallschmelzen unterschiedlich sein können.

Als Trägergas können Inertgase verwendet werden, vorzugsweise Argon und/oder Stickstoff. Als beigemischtes Gas kann Kohlenmomoxid verwendet werden, so dass der KohlenmonoxidGehalt in der Metallschmelze gemessen werden kann.

Ein Ausführungsbeispiel der Erfindung wird nachfolgend an Hand einer Zeichnung näher erläutert.

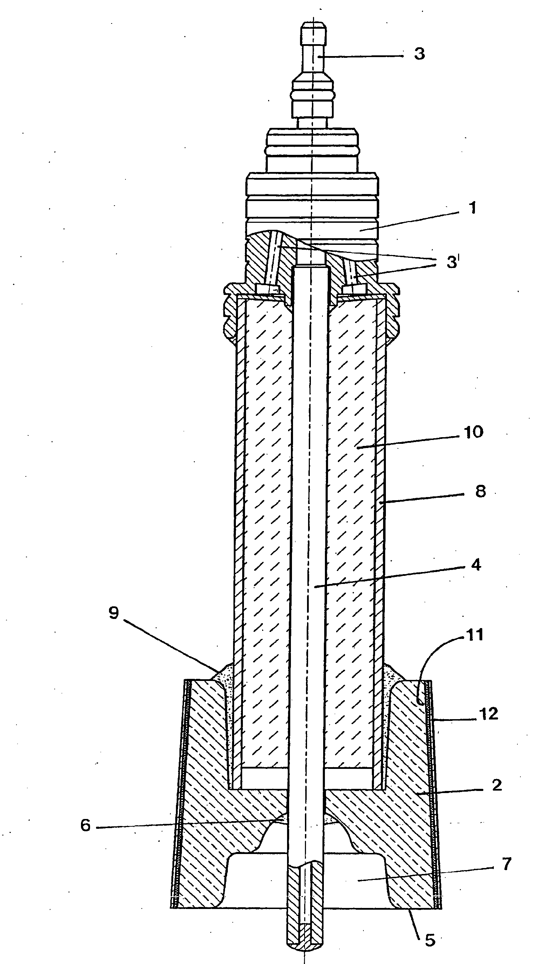

Die Zeichnung zeigt eine erfindungsgemäße Vorrichtung, teilweise geschnitten.

Die in der Zeichnung dargestellte Vorrichtung wird an einem Befestigungsstutzen 1 an einen nicht dargestellten Trägerrohr befestigt und mit diesem in eine Stahlschmelze eingetaucht. In die Stahlschmelze wird der Gassammelkörper 2 getaucht, um dort den Gasaustausch vorzunehmen.

In dem Befestigungsstutzen 1 sind Gasanschlüsse 3; 3' angeordnet. Dabei mündet der zentrale Gasanschluss 3 in die zentrisch in der Vorrichtung angeordnete Gaszuleitung 4. Diese ist zentrisch durch den Gassammelkörper hindurch geführt und endet unterhalb der Stirnseite 5 des Gassammelkörpers. Durch die Gaszuleitung 4 wird Trägergas in die Metallschmelze eingeleitet. Die Gaszuleitung 4 besteht im Wesentlichen aus einem Quarzrohr, welches an seinem Eintauchende gebogen sein kann, so dass die Mündungsöffnung in Richtung des Gassammelkörpers 2 gerichtet ist. Die Gaszuleitung 4 ist in dem Gassammelkörper 2 mittels Zement 6 fixiert. Das durch die Gaszuleitung 4 in die Metallschmelze einströmende Trägergas nimmt aus der Metallschmelze Gase auf, steigt in den Hohlraum 7 des Gassammelkörpers 2 und dringt von dort und von der Stirnseite 5 her in den Gassammelkörper 2 ein. Dieser ist aus einem porösen Material gebildet, beispielsweise aus Zement. Auch ein Keramikkörper, beispielsweise Aluminiumoxid, ist möglich. Durch die Poren des Gassammelkörpers dringt das Gas nach oben in die Gasableitung. Diese ist im Wesentlichen gebildet aus einem Quarzglasrohr 8, welches mittels Zement 9 in dem Gassammelkörper 2 fixiert ist. In dem Quarzglasrohr ist eine poröse Füllung 10 aus Aluminiumoxid, zum Beispiel in Kugelform, angeordnet. Durch die Füllung 10 erfolgt die Ableitung des mit Gas aus der Metallschmelze gemischten Trägergases durch die Gasanschlüsse 3' zu einer Messeinrichtung. Dort wird das entnommene Gas mit dem in die Metallschmelze eingeleiteten Gas verglichen und so das aus der Schmelze aufgenommene (oder abgegebene) Gas ausgewertet und dadurch der Gasgehalt in der Metallschmelze bestimmt. Dieser Vorgang ist an sich hinreichend bekannt und beispielsweise in EP 307 430 B1 (oder ähnlich in EP 563 447 A1) beschrieben. Als Trägergas des eingeleiteten Gases wird Argon verwendet. Dem Trägergas wird zur Messung des Kohlenmonoxidgehaltes in der Stahlschmelze jeweils Kohlenmonoxid mit einem Anteil von jeweils mehr als 2,5 % (beispielsweise von 5 % und 10 %) beigemischt, da der erwartete Gasgehalt bei 2,5 % liegt.

Der Gassammelkörper 2 weist an seiner konischen Außenfläche eine aus einer unteren Teilschicht 11 und einer äußeren Teilschicht 12 bestehende gasundurchlässige Schicht auf. Die untere Teilschicht 11 ist aus Molybdän gebildet, die äußere Teilschicht 12 dient als Schutzschicht und ist aus Spinell gebildet.

Prinzipiell kann die gasundurchlässige Schicht auch an dem dem Eintauchende angewandten Ende des Gassammelkörpers 2 angeordnet sein. Dies ist jedoch im Regelfall nicht nötig, da die dort vorhandenen Oberflächen derart klein sind, dass ein Gasaustritt nur in nicht nennenswertem Umfang erfolgt. Dadurch wird praktisch das gesamte von der Vorrichtung aufgenommene Gas in die von dem Quarzglasrohr 8 begrenzte Gasableitung geleitet.

Mit der Vorrichtung kann auch der Gehalt an Wasserstoff oder Stickstoff in Stahlschmelzen bestimmt werden.

## Patentansprüche

1. Vorrichtung zum Sammeln von Gasen in Metallschmelzen mit einem einen Gassammelkörper (2) aufweisenden Eintauchende, einer an dem Eintauchende mündenden Gaszuleitung (4) und einer Gasableitung für die den Gassammelkörper (2) durchdringenden Gase, wobei der Gassammelkörper (2) eine am Eintauchende angeordnete Stirnseite (5) und Seitenwände aufweist, wobei der Gassammelkörper (2) selbst undurchlässig für die Metallschmelze ist, wobei der Gassammelkörper (2) aus porösem Material gebildet ist, so dass durch die Poren des Gassammelkörpers (2) das Gas in die Gasableitung dringt , **dadurch gekennzeichnet, dass** eine gasundurch- lässige Schicht (11;12) an der Oberfläche der Seitenwände des Gassammelkörpers (2) angeordnet ist.

2. Vorrichtung nach Anspruch 1, **dadurch gekennzeichnet, dass** die Schicht (11;12) aus mindestens zwei aufeinander angeordneten Teilschichten gebildet ist.

3. Vorrichtung nach Anspruch 2, **dadurch gekennzeichnet, dass** eine untere, dem Inneren des Gassammelkörpers (2) zugewandte Teilschicht (11) aus Metall gebildet ist.

4. Vorrichtung nach Anspruch 3, **dadurch gekennzeichnet, dass** die untere Teilschicht (11) aus einem Metall mit höherem Schmelzpunkt als Eisen gebildet ist.

5. Vorrichtung nach Anspruch 3, **dadurch gekennzeichnet, dass** die untere Teilschicht (11) im Wesentlichen aus einem Metall der Gruppe Molybdän, Titan, Vanadium, Chrom, Niob oder einer Legierungen mit mindestens einem dieser Metalle gebildet ist.

6. Vorrichtung nach einem der Ansprüche 2 bis 5, **dadurch gekennzeichnet, dass** eine äußere, dem Inneren des Gassammelkörpers (2) abgewandte Teilschicht (12) aus Keramik gebildet ist.

7. Vorrichtung nach Anspruch 6, **dadurch gekennzeichnet, dass** die äußere Teilschicht (12) aus Oxidkeramik oder einem Silikat gebildet ist.

8. Vorrichtung nach Anspruch 7, **dadurch gekennzeichnet, dass** die äußere Teilschicht (12) aus Zirkoniumdioxid, Aluminiumoxid, Chromdioxid, Zirkoniumsilikat, Aluminiumsilikat oder Spinell gebildet ist.

9. Vorrichtung nach einem der Ansprüche 1 bis 8, **dadurch gekennzeichnet, dass** mindestens eine Teilschicht plasmagespritzt ist.

10. Vorrichtung nach einem der Ansprüche 1 bis 9, **dadurch gekennzeichnet, dass** der Gassammelkörper (2) eine zylindrische oder konische Seitenwand aufweist.

11. Vorrichtung nach einem der Ansprüche 1 bis 10, **dadurch gekennzeichnet, dass** die Gasableitung an der der Stirnseite (5) gegenüberliegenden Rückwand des Gassammelkörpers (2) angeordnet ist.

12. Vorrichtung nach einem der Ansprüche 1 bis 11, **dadurch gekennzeichnet, dass** die Gasableitung an einem Gasanschlussstutzen oder in einer Öffnung des Gassammelkörpers (2) angeordnet ist.

13. Verwendung der Vorrichtung nach einem der Ansprüche 1 bis 12 zum Messen des Gasgehaltes in einer Metallschmelze.

## Claims

1. Device for collecting gases in melted metals having an immersion end comprising a gas collecting body (2), a gas feed line (4) terminating at the immersion end and a gas discharge line for the gases permeating through the gas collecting body (2), whereby the gas collecting body (2) comprises a front side (5) arranged on the immersion end, and side walls, whereby the gas collecting body (2) itself is impermeable to the melted metal, whereby the gas collecting body (2) is made of porous material such that the gas penetrates into the gas discharge line through the pores of the gas collecting body (2), **characterised in that** a gas-impermeable layer (11; 12) is arranged on the surface of the side walls of the gas collecting body (2).

2. Device according to claim 1, **characterised in that** the layer (11; 12) is made of at least two part-layers arranged on top of each other.

3. Device according to claim 2, **characterised in that** a lower part-layer (11) facing the inside of the gas collecting body (2) is made of metal.

4. Device according to claim 3, **characterised in that** the lower part-layer (11) is made of a metal that has a melting point higher than iron.

5. Device according to claim 3, **characterised in that** the lower part-layer (11) is essentially made of a metal from the group of molybdenum, titanium, vanadium, chromium, niobium or an alloy comprising at least one of said metals.

6. Device according to any one of the claims 2 to 5, **characterised in that** an outer part-layer (12) facing away from the inside of the gas collecting body (2) is made of ceramic material.

7. Device according to claim 6, **characterised in that** the outer part-layer (12) is made of oxide ceramics or of a silicate.

8. Device according to claim 7, **characterised in that** the outer part-layer (12) is made of zirconium dioxide, aluminium dioxide, chromium dioxide, zirconium silicate, aluminium silicate or spinel.

9. Device according to any one of the claims 1 to 8, **characterised in that** at least one part-layer is plasma-sprayed.

10. Device according to any one of the claims 1 to 9, **characterised in that** the gas collecting body (2) comprises a cylindrical or conical side wall.

11. Device according to any one of the claims 1 to 10, **characterised in that** the gas discharge line is arranged on the rear wall of the gas collecting body (2) that is situated opposite from the front side (5).

12. Device according to any one of the claims 1 to 11, **characterised in that** the gas discharge line is arranged at a gas connection socket or in an opening of the gas collecting body (2).

13. Use of the device according to any one of the claims 1 to 12 for measuring the gas content in a melted metal.

## Revendications

1. Dispositif destiné à la collecte de gaz dans du métal en fusion comprenant une extrémité plongeante présentant un corps de collecte des gaz (2), une arrivée de gaz (4) débouchant sur l'extrémité plongeante et une évacuation de gaz pour les gaz traversant le corps de collecte des gaz (2), sachant que le corps de collecte des gaz (2) présente une face avant (5) disposée sur l'extrémité plongeante et des parois latérales, sachant que le corps de collecte des gaz (2) est lui-même imperméable pour le métal en fusion, sachant que le corps de collecte des gaz (2) est fabriqué dans un matériau poreux, de telle sorte que le gaz pénètre dans l'évacuation de gaz par les pores du corps de collecte des gaz (2), **caractérisé en ce qu'**une couche imperméable aux gaz (11; 12) est disposée à la surface des parois latérales du corps de collecte des gaz (2).

2. Dispositif selon la revendication 1, **caractérisé en ce que** la couche (11; 12) est formée d'au moins deux couches partielles disposées l'une sur l'autre.

3. Dispositif selon la revendication 2, **caractérisé en ce qu'**une couche partielle (11) inférieure, tournée vers l'intérieur du corps de collecte des gaz (2) est fabriquée en métal.

4. Dispositif selon la revendication 3, **caractérisé en ce que** la couche partielle (11) inférieure est fabriquée dans un métal ayant un point de fusion plus élevé que le fer.

5. Dispositif selon la revendication 3, **caractérisé en ce que** la couche partielle (11) inférieure est fabriquée essentiellement dans un métal du groupe molybdène, titane, vanadium, chrome, niobium ou d'alliages comprenant au moisn l'un de ces métaux.

6. Dispositif selon l'une des revendications 2 à 5, **caractérisé en ce qu'**une couche partielle (12) extérieure, détournée de l'intérieur du corps de collecte des gaz (2), est fabriquée en céramique.

7. Dispositif selon la revendication 6, **caractérisé en ce que** la couche partielle (12) extérieure est fabriquée en céramique oxydée ou dans un silicate.

8. Dispositif selon la revendication 7, **caractérisé en ce que** la couche partielle (12) extérieure est fabriquée en dioxyde de zirconium, oxyde d'aluminium, dioxyde de chrome, silicate de zirconium, silicate d'aluminium ou en spinelle.

9. Dispositif selon l'une des revendications 1 à 8, **caractérisé en ce qu'**au moins une couche partielle est pulvérisée par plasma.

10. Dispositif selon l'une des revendications 1 à 9, **caractérisé en ce que** le corps de collecte des gaz (2) présente une paroi latérale cylindrique ou conique.

11. Dispositif selon l'une des revendications 1 à 10, **caractérisé en ce que** l'évacuation de gaz est disposée sur la paroi arrière du corps de collecte des gaz (2) opposée à la face avant (5).

12. Dispositif selon l'une des revendications 1 à 11, **caractérisé en ce que** l'évacuation de gaz est disposée sur une tubulure de raccordement du gaz ou dans une ouverture du corps de collecte des gaz (2).

13. Emploi d'un dispositif selon l'une des revendications 1 à 12 pour mesurer la teneur en gaz dans du métal en fusion.
